# EUROPEAN PATENT APPLICATION

(11) **EP 3 795 584 A1**
(43) Date of publication of application: **24.03.2021**
(21) Application number: 19733942.7
(22) Date of filing: 16.05.2019
(51) Int. Cl.: C07K 14/59, A61K 38/24

(54) **RECOMBINANT BOVINE FOLLICLE-STIMULATING HORMONE, COMPOSITION COMPRISING THE SAME, AND METHOD TO INDUCE SUPEROVULATION AND ESTRUS SYNCHRONIZATION IN BOVINES USING SAID HORMONE**

(30) Priority: 18.05.2018 CL 20181347
(71) Applicant: Centro de Biotecnología y Biomedicina SPA, Concepción (CL); Fundaçao Edson Quiroz - Universidad de Fortaleza, 60811-905 Fortaleza (BR)
(72) Inventor: SANCHEZ RAMOS, Oliberto, Concepción (CL); TOLEDO ALONSO, Jorge, Concepción (CL); HUGUES SALAZAR, Florence, Chillán (CL); CABEZAS ÁVILA, Ignacio, San Nicolás (CL); CERRO GARRIDO, Rita Paulina, Concepción (CL); PARRA PEREIRA, Natalie Carolina, Concepción (CL); ACOSTA ALBA, Jannel, Concepción (CL); SIMIANO TAVARES, Kaio Cesar, 60130-271 Fortaleza (BR); TONDELLO MARTINS, Leonardo, 60811-120 Fortaleza (BR); GAUDENCIO NETO, Saul, 60160-110 Fortaleza (BR)
(74) Representative: Herrero & Asociados, S.L.
(86) International application number: PCT/CL2019/050040
(87) International publication number: WO 2019/218096

(57) **Abstract**

The present invention relates to the technical field of Biotechnology and the obtaining of molecules by recombinant DNA technology, in particular it refers to a synthetic polynucleotide sequence encoding a single-chain recombinant bovine follicle-stimulating hormone, comprising a first polynucleotide enconding the beta subunit of the *Bos taurus* follicle-stimulating hormone having sequence SEQ ID No. 1, or a variant thereof with at least 85% sequence identity therewith; a second polynucleotide encoding the alpha subunit of the *Bos taurus* follicle-stimulating hormone having sequence SEQ ID No. 2 or a variant thereof with at least 85% sequence identity therewith; and a third polynucleotide linking said first and second polynucleotides, which encodes a spacer sequence derived from human keratin 10 having sequence SEQ ID No. 3 or a variant. The invention also includes the genetic construct including these sequences, the cells transformed therewith, and the pharmaceutical composition comprising said single-chain recombinant bovine follicle-stimulating hormone.

## Description

### Technical Field

The present invention relates to the technical field of recombinant peptides production, particularly biologically active hormones, such as the bovine follicle-stimulating hormone. Additionally, it relates to compositions for veterinary use, particularly with a composition comprising the recombinant bovine follicle-stimulating hormone, and its use for increasing reproduction in bovines and other ungulates.

### Background

In the agricultural and livestock industry, improvements in reproductive science and the multiplication of more productive genotypes are directly linked to increases in productivity, which is why it is particularly important to increase reproductive performance in animals of commercial interest, particularly, bovines and ovines. One of way to increase the reproductive performance of these animals is to induce superovulation of females with a desirable genotype. To this end, a hormone treatment with follicle-stimulating hormone (FSH) is used, which induces the ovulation of several oocytes when injected into females. FSH is a glycoprotein hormone that is a heterodimer, a beta, specific for FSH, and an alpha, common to luteinizing hormone (LH) and others.

Currently, FSH is extracted from the pituitary glands of slaughtered pigs, where injectable hormones having this origin such as Folltropin-V™ and Pluset™ are nowadays the most used by the livestock industry. Due to its origin and structure, these exogenous hormones have a number of risks associated with their use, including the risk of contamination with pathogens such as prions or microorganisms, contamination of the hormonal preparation containing LH, since it is purified together with the FSH, and some preparations even declare this effect. The effect of said contamination reduces the process of superovulation, reduces the efficiency of fertilization, and affects the quality of embryos. In addition, due to their animal origin, these hormonal preparations have a circulation half-life of about 5 hours, which requires a considerable amount of injections during the superovulation protocol. Additionally, having a porcine origin, this hormone can induce a humoral immune response when administered in other species, such as bovines.

Due to all the aforementioned problems, the livestock industry has sought to improve the quality of the FSH administered, mainly, by means of molecular techniques where FSH of recombinant origin has been generated. In the state of the art, different alternatives of recombinant bovine FSH have been proposed. Document WO 2008/098169 A2 refers to the production of a recombinant bovine FSH (BoviPure FSH™) where the α subunit is fused with the β subunit using a spacer sequence to generate a unique DNA sequence that encodes this recombinant protein. The spacer sequence corresponds to the Carboxy-terminal peptide of human chorionic gonadotropin (CTP), which generates a linear sequence. Consequently, they generate a recombinant FSH, where it is not explicit whether there is any increase in the half-life of this protein or another similar advantage. The use of CTP as a spacer sequence to generate a recombinant FSH, is also disclosed in document US 2015/0335713 A1, which refers to a method to increase the reproductive performance of female bovines with respect to superovulation and generation of viable embryos using recombinant FSH. This recombinant FSH was generated from the fusion of the β subunit with the α subunit of bovine FSH using CTP as a spacer peptide or repeated G4S in a single amino acid chain.

In the general disclosure document of 2005 (Ruman JI, Pollak S, Trousdale RK, Klein J, LustbaderJW. Effects of long-acting recombinant human follicle-stimulating hormone analogs containing N-linked glycosylation on murine folliculogenesis. Fertil Steril. 2005 Apr; 83 Suppl 1: 1303-9), the generation of recombinant FSH in a single sequence by the use of the CTP spacer peptide is also promoted.

In the state of the art, in order to increase the half-life and biological activity of FSH, site-directed mutagenesis has been used in both the alpha and beta units of the FSH, as shown in document US 5,338,835, which refers to the production of recombinant FSH, among others, by the generation of a fusion protein where the α subunit common to FSH and LH fuses with the β subunit of FSH, which in turn is coupled in its carboxyl-terminal (C-Ter) to a peptide of 34 CTP amino acids, as a spacer peptide, generating a linear sequence. In this document, recombinant FSH versus native FSH in rats is tested, showing that recombinant FSH has a longer half-life than native FSH.

Also, recombinant FSH have been generated by co-transfection of the alpha and beta subunit in human cell lines such as HEK293, as disclosed in the document WO 2014/183175 A1, which refers to the development of a method for production and/or purification of hormones, focusing on FSH from human or animal origin, using human cells as cell lines for the expression of the same. This recombinant FSH was tested in rats, showing that the recombinant FSH increases the weight of the ovary of the rat after a daily injection of recombinant FSH with human chorionic gonadotropin hormone for a period of 3 days.

In the article of Gifre, L. *et al.*, 2017 (Gifre, L., Arís, A., Bach, A., & Garcia-Fruitos, E. Trends in recombinant protein use in animal production. Microbial cell factories. 2017, 16(1), 40.), a comprehensive study was conducted on the market of different hormonal supplements, among them, native and recombinant FSH, showing that the current objective of the market is to seek for a long-lasting recombinant FSH, for its use in the agricultural and livestock market.

Therefore, in order to reduce the number of problems of the porcine FSH that is currently on the market, there is a need to generate a new, improved recombinant FSH, with a longer half-life, to reduce the number of injections and the cost associated thereto.

### Summary of the invention

A first object of the present invention is a synthetic polynucleotide sequence that encodes a single-chain recombinant bovine follicle-stimulating hormone, comprising;
(i) a first polynucleotide encoding the beta subunit of the follicle-stimulating hormone of the *Bos taurus* species having sequence SEQ ID No. 1, or a variant thereof with at least 85% sequence identity therewith;
(ii) a second polynucleotide encoding the alpha subunit of the follicle-stimulating hormone of the *Bos taurus* species having sequence SEQ ID No. 2 or a variant thereof with at least 85% sequence identity therewith; and
(iii) a third polynucleotide linking said first and second polynucleotides, which encodes a spacer sequence derived from human keratin 10 having sequence SEQ ID No. 3 or a variant thereof with at least 85% sequence identity therewith.

The first mentioned synthetic polynucleotide sequence encoding the beta subunit of the follicle-stimulating hormone of the *Bos taurus* species having sequence SEQ ID No. 1, includes at its 5' end a sequence encoding an endogenous secretion signal. On the other hand, the second polynucleotide encoding the alpha subunit of the follicle-stimulating hormone of the *Bos taurus* species having sequence SEQ ID No. 2, includes at its 3' end a sequence encoding a multiple histidine region.

In a preferred embodiment, the synthetic polynucleotide sequence encoding a single-chain recombinant bovine follicle-stimulating hormone is the sequence identified as SEQ ID No. 4.

A second object of the present invention is a vector for the expression of a synthetic polynucleotide sequence encoding a single-chain recombinant bovine follicle-stimulating hormone, comprising a DNA construct containing a promoter sequence operatively linked to:
(i) a first polynucleotide encoding the beta subunit of the follicle-stimulating hormone of the *Bos taurus* species having sequence SEQ ID No. 1, or a variant thereof with at least 85% sequence identity therewith;
(ii) a second polynucleotide encoding the alpha subunit of the follicle-stimulating hormone of the *Bos taurus* species having sequence SEQ ID No. 2 or a variant thereof with at least 85% sequence identity therewith; and
(iii) a third polynucleotide linking said first and second polynucleotides, which encodes a spacer sequence derived from human keratin 10 having sequence SEQ ID No. 3 or a variant thereof with at least 85% sequence identity therewith; and
(iv) a cleavage and polyadenylation sequence.

Particularly, in a preferred embodiment the aforementioned vector is a lentiviral vector.

In a preferred embodiment, the vector for the expression of a synthetic polynucleotide sequence encoding a single-chain recombinant bovine follicle-stimulating hormone, comprises a DNA construct containing a promoter sequence operatively linked to SEQ ID No. 4 and a cleavage and polyadenylation sequence.

A third object of the present invention is a transduced cell expressing a single-chain recombinant bovine follicle-stimulating hormone, comprising a vector for the expression of a synthetic polynucleotide sequence encoding said single-chain recombinant bovine follicle-stimulating hormone, wherein said vector comprises a DNA construct that contains a promoter sequence operatively linked to:
(i) a first polynucleotide encoding the beta subunit of the *Bos taurus* follicle-stimulating hormone having sequence SEQ ID No. 1, or a variant thereof with at least 85% sequence identity therewith;
(ii) a second polynucleotide encoding the alpha subunit of the *Bos taurus* follicle-stimulating hormone having sequence SEQ ID No. 2 or a variant thereof with at least 85% sequence identity therewith; and
(iii) a third polynucleotide linking said first and second polynucleotides, which encodes a spacer sequence derived from human keratin 10 having sequence SEQ ID No. 3 or a variant thereof with at least 85% sequence identity therewith; and
(iv) a cleavage and polyadenylation sequence.

In a preferred embodiment, the transduced cell is a Chinese hamster ovary cell.

In a preferred embodiment, the cell transduced with the vector for the expression of a synthetic polynucleotide sequence encoding a single-chain recombinant bovine follicle-stimulating hormone, comprises a DNA construct containing a promoter sequence operatively linked to SEQ ID No. 4 and a cleavage and polyadenylation sequence.

A fourth object of the present invention is a synthetic polypeptide sequence of a single-chain recombinant bovine follicle-stimulating hormone, comprising:
(i) a first polypeptide encoding the beta subunit of the *Bos taurus* follicle-stimulating hormone having sequence SEQ ID No. 5, or a variant thereof with at least 85% sequence identity therewith;
(ii) a second polypeptide encoding the alpha subunit of the *Bos taurus* follicle-stimulating hormone having sequence SEQ ID No. 6 or a variant thereof with at least 85% sequence identity therewith; and
(iii) a third polypeptide linking said first and second polypeptides, which is a spacer sequence derived from human keratin 10 having sequence SEQ ID No. 7 or a variant thereof with at least 85% sequence identity therewith, comprising at least 2 N-glycosylation sites.

Preferably, the first polypeptide having sequence SEQ ID No. 5 includes at its amino terminal end a signal sequence of endogenous secretion, while the second polypeptide having sequence SEQ ID No. 6 includes at its carboxyl terminal end a multiple histidine region.

In a preferred embodiment, the synthetic polypeptide sequence of a single-chain recombinant bovine follicle-stimulating hormone is the sequence SEQ ID No. 8.

A fifth object of the present invention is a method for obtaining a single-chain recombinant bovine follicle-stimulating hormone, comprising the steps of:
- providing a synthetic polynucleotide sequence encoding a single-chain recombinant bovine follicle-stimulating hormone, comprising:
   (i) a first polynucleotide encoding the beta subunit of the *Bos taurus* follicle-stimulating hormone having sequence SEQ ID No. 1, or a variant thereof with at least 85% sequence identity therewith;
   (ii) a second polynucleotide encoding the alpha subunit of the *Bos taurus* follicle-stimulating hormone having sequence SEQ ID No. 2 or a variant thereof with at least 85% sequence identity therewith; and
   (iii) a third polynucleotide linking said first and second polynucleotides, which encodes a spacer sequence derived from human keratin 10 having sequence SEQ ID No. 3 or a variant thereof with at least 85% sequence identity therewith;
- cloning said synthetic polynucleotide sequence into an expression vector comprising a DNA construct containing a promoter sequence operatively linked to the synthetic polynucleotide sequence, a cleavage and polyadenylation sequence;
- transducing cells with said expression vector and culturing said transduced cells in a culture medium;
- obtaining said culture medium containing the synthetic polypeptide sequence of the single-chain recombinant bovine follicle-stimulating hormone produced by the transduced cells; and
- purifying the single-chain recombinant bovine follicle-stimulating hormone from the culture medium.

Preferably, the synthetic polynucleotide sequence encoding the single-chain recombinant bovine follicle-stimulating hormone is SEQ ID No. 4., and the synthetic polypeptide sequence of the single-chain recombinant bovine follicle-stimulating hormone is SEQ ID No. 8.

In another preferred embodiment, the expression vector used in the aforementioned method is a lentiviral vector.

Preferably, the transduced cells are Chinese hamster ovary cells, in a medium preferably free of serum and free of proteins.

The purification of the synthetic polypeptide sequence of the single-chain recombinant bovine follicle-stimulating hormone is performed, in a preferred embodiment, by means of affinity chromatography columns.

A sixth object of the present invention is a pharmaceutical composition containing a synthetic polynucleotide of a single-chain recombinant bovine follicle-stimulating hormone, wherein said hormone has an amino acid sequence comprising:
(i) a first polypeptide encoding the beta subunit of the *Bos taurus* follicle-stimulating hormone having sequence SEQ ID No. 5, or a variant thereof with at least 85% sequence identity therewith;
(ii) a second polypeptide encoding the alpha subunit of the *Bos taurus* follicle-stimulating hormone having sequence SEQ ID No. 6 or a variant thereof with at least 85% sequence identity therewith; and
(iii) a third polypeptide linking said first and second polypeptides, which is a spacer sequence derived from human keratin 10 having sequence SEQ ID No. 7 or a variant thereof with at least 85% sequence identity therewith, comprising at least 2 N-glycosylation sites; and
pharmaceutically acceptable excipients.

Preferably, the pharmaceutical composition comprises the synthetic polypeptide sequence of the single-chain recombinant bovine follicle-stimulating hormone of SEQ ID No. 8.

A final object of the present invention is a method of treatment for the superovulation of ungulates, comprising:
- the administration of a dose of a pharmaceutical composition comprising:
   (i) a first polypeptide encoding the beta subunit of the *Bos taurus* follicle-stimulating hormone having sequence SEQ ID No. 5, or a variant thereof with at least 85% sequence identity therewith;
   (ii) a second polypeptide encoding the alpha subunit of the *Bos taurus* follicle-stimulating hormone having sequence SEQ ID No. 6 or a variant thereof with at least 85% sequence identity therewith; and
   (iii) a third polypeptide linking said first and second polypeptides, which is a spacer sequence derived from human keratin 10 having sequence SEQ ID No. 7 or a variant thereof with at least 85% sequence identity therewith, comprising at least 2 N-glycosylation sites;
- wherein said pharmaceutical composition is administered every 12 hours, for 72 hours.

Preferably, the pharmaceutical composition comprises the synthetic polypeptide sequence of the single-chain recombinant bovine follicle-stimulating hormone of SEQ ID No. 8. Accordingly, preferably, the total dose administered ranges from 100 to 1000 µg of the synthetic polypeptide sequence of the single-chain recombinant bovine follicle-stimulating hormone of SEQ ID No. 8.

### Brief description of the Figures

FIG. 1 shows an image of the polypeptide sequence of the single-chain recombinant bovine FSH.
FIG. 2 shows an image of the three-dimensional structure of the single-chain recombinant bovine FSH protein.
FIG. 3 shows the map of the expression vector of the single-chain recombinant bovine FSH (scbFSH in the map) of the present invention.
FIG. 4 shows the detailed expression cassette of the single-chain recombinant bovine scbFSH, as well as the entire sequence of molecular events required for the detection and selection of high-producer cell clones of scbFSH.
FIG. 5 Shows the general working procedure followed for obtaining clones of scbFSH-producing CHO cells.
FIG. 6 Shows a *Western blot* where the expression of scbFSH is detected from four preselected producer clones. In each lane, 100 microliters of conditioned culture medium of each of the clones was applied.
FIG. 7 shows a purification chromatogram of single-chain recombinant bovine FSH, where NoB corresponds to the unbound protein, W corresponds to the protein peak after column washing, and E corresponds to the protein peak after adding the elution buffer.
FIG. 8 shows an SDS-PAGE electrophoresis (A) and a *Western blot* (B) of scbFSH with the results of the purification thereof, where In is the input sample (culture medium with bovine simple chain recombinant FSH), NoB corresponds to the unbound protein, W (washing) corresponds to the protein peak after column washing, E corresponds to the protein peak after adding the elution buffer.
FIG. 9 shows the profile of oligosaccharides N-linked to the purified simple bovine recombinant FSH molecule. **(A)** Relative abundance of total oligosaccharides N-linked to the purified glycoprotein. **(B)** Oligosaccharides treated with the neuraminidase enzyme, which removes the terminal sialic acid monosaccharide.
FIG. 10 shows the comparison of the single-chain recombinant bovine FSH purity analysis with respect to the commercial FSH Folltropin-V®.
FIG. 11 shows the assay result of an *in vitro* maturation of bovine oocytes with single-chain recombinant bovine FSH. **(A)** Maturation medium without FSH, **(B)** maturation medium containing 1 µg / mL of FSH Folltropin-V®, **(C)** maturation medium containing 0.01 IU / mL Pluset, **(D)** maturation medium that contains 1.5 ng / mL of single-chain recombinant bovine FSH.
FIG. 12 is an illustrative image of the follicles detected by means of transrectal ultrasonography in bovines treated with single-chain recombinant bovine FSH.

### Detailed description of the invention

The present invention relates to a synthetic nucleotide sequence encoding a bovine recombinant follicle-stimulating hormone, a synthetic polypeptide sequence of said bovine recombinant follicle-stimulating hormone, a pharmaceutical composition comprising the same, and its use to increase reproduction in bovines and other ungulates. The advantage of the present recombinant hormone is its long half-life demonstrated *in vivo*, particularly in bovines, which also have a biological response significantly superior to the response that is usually obtained with commercially available hormones to date.

All the technical and scientific terms used to describe the present invention have the same meaning as is understood for a person with basic knowledge in the technical field in question. However, to define more clearly the scope of the invention as defined by the claims, a list of the terms used in this description is included below.

The term "FSH" should be understood as the acronym in English of Follicle-Stimulating Hormone, as a glycoproteic polypeptide that is produced in the pituitary gland whose half-life in its natural state is between 2 to 4 hours. This hormone stimulates follicular growth and development, and it is commonly used to induce superovulation in ungulates.

The term "alpha subunit" corresponds to the alpha subunit of the heterodimer corresponding to the follicle-stimulating hormone, which is common to several proteins such as the luteinizing hormone (LH) or other hormones. In turn, the term "beta subunit" corresponds to the beta subunit of the heterodimer corresponding specifically to the follicle-stimulating hormone.

The term "nucleotide sequence" or "polynucleotides", should be understood as a double strand of DNA, or a single strand of DNA, natural or synthetic, or products of the transcription of said DNA (for example, RNA molecules). In turn, a "DNA sequence encoding a product of interest", should be understood as a nucleotide sequence that transcribes a functional RNA molecule, or that encodes a peptide, polypeptide, or functional protein of interest to the present invention. It should be understood that the present invention is not related to genomic nucleotide sequences in their natural state, but refers to nucleotide sequences in an isolated, or purified, or partially purified, synthetic or recombinant state, obtained by any method of genetic engineering known in the state of the art.

The term "peptide sequence" or "polypeptides" should be understood as a sequence of amino acids, natural or synthetic, or products of RNA translation. When these polypeptides have a stable and three-dimensional structure, they are called proteins.

The term "recombinant" refers to any nucleotide (DNA, RNA) or amino acid sequence modified by any method of genetic engineering known in the state of the art, which generates, as a result, a new nucleotide or amino acid sequence different from that found in nature.

The term "identity" or "similarity" between nucleotide sequences should be understood as the percentage of identical nucleotides that the compared sequences share with each other, in a particular comparison window. The percentage of identity can be calculated by using a sequence comparison algorithm or by manual alignment and visual inspection. For example, sequences and identity percentages can be obtained by using computer resources available on the Internet, such as the computer program BLAST (http://blast.ncbi.nlm.nih.gov/) or the computer program FastDB. The identity of sequences can also be determined by means of hybridization assays thereof. The greater the degrees of hybridization astringency used in the assay, the greater the complementarity of the sequences required for them to hybridize. High stringency conditions are described by Sambrook et al. (Molecular Cloning A Laboratory Manual, Cold Spring Harbor Press, 1989).

The term "DNA construct" or "expression cassette" should be understood as a nucleotide sequence artificially created by genetic engineering tools, which generally comprises a transcription promoter sequence, operatively linked to a DNA sequence encoding a protein of interest, and adjacent to it a DNA cleavage and polyadenylation sequence. For this DNA construct to be expressed in an appropriate host, in the case of the present invention, a eukaryotic cell, or a higher organism, must be inserted into an expression vector, which contains all of the DNA sequences necessary for the proper expression within said host, together with a series of molecular markers that allow to detect and select those hosts that have been transformed.

The term "spacer sequence" corresponds to a sequence of nucleotides that binds gene sequences encoding multimeric proteins that are bound by non-covalent bonds in nature. This way, the spacer sequence makes it possible to obtain a single-chain multimeric protein without interfering with the biological activity of said protein. The term "spacer sequence" may also refer to the peptide sequence that is encoded by the polynucleotide spacer sequence. In particular for the present invention, the spacer sequence links the beta and alpha sequences of the bovine recombinant follicle-stimulating hormone, maintaining the steric folding properties of both subunits without interfering in the binding of this molecule to the receptor, thus generating a sole amino acid chain.

The term "transduced cell" should be understood as a host cell subject to the transduction process by which an exogenous DNA sequence is incorporated. Said cell can be a eukaryotic somatic cell or an embryo.

The term "ungulates" refer to placental mammals that sustain themselves and walk with the ends of the fingers, that may or may not be covered by a hoof. Some examples of ungulates are animals for cattle such as bovines, ovines, caprines, porcines, equines, deer, among others, not limited to those previously mentioned.

The term "superovulation" should be understood as the increased number of ovulated follicles or the production of multiple embryos by the injection of FSH.

The term "effective amount" should be understood as the amount of FSH that must be administered to produce the desired effect.

As previously described, a first object of the present invention relates to a synthetic polynucleotide sequence that encodes a single-chain recombinant bovine FSH. This sequence was designed without altering the three-dimensional structure of the polypeptide sequence encoded in the synthetic polynucleotide sequence. In order to obtain said sequence that encodes the single-chain recombinant bovine FSH, the alpha chain was linked to the beta chain of the FSH, in such a way that the linking met two fundamental requirements: (1) that both subunits retained sufficient steric freedom so as not to affect its folding and assembly capacity, as it occurs naturally with the heterodimer, and (2) that the peptide resulting from the linker sequence will not affect the interaction of the binding of the recombinant FSH with its receptor. Based on these requirements, a synthetic polynucleotide sequence encoding a single-chain recombinant bovine follicle-stimulating hormone was obtained, comprising: (i) a first polynucleotide encoding the beta subunit of the follicle-stimulating hormone of the *Bos taurus* species having sequence SEQ ID No.1, or a variant thereof with at least 85% sequence identity therewith; covalently linked to (ii) a second polynucleotide encoding the alpha subunit of the follicle-stimulating hormone of the *Bos taurus* species having sequence SEQ ID No. 2 or a variant thereof with at least 85% sequence identity therewith; and covalently linked to (iii) a third polynucleotide linking said first and second polynucleotides, which encodes a spacer sequence derived from human keratin 10 having sequence SEQ ID No. 3 or a variant thereof with at least 85% identity of sequence therewith. As these three sequences or their variants are covalently linked, a configuration of the subunit type of the FSH (beta) -linker- (alpha) is formed.

While it is mentioned that, preferably, SEQ ID No. 1 corresponds to the beta subunit of the beta subunit sequence of the *Bos taurus* FSH, the present invention is not limited to the use of said sequence and the sequence of the beta subunit of any other ungulate may be used. Similarly, while the preferred embodiment of the invention is SEQ ID No. 2, corresponding to the alpha subunit of the alpha subunit sequence of the *Bos Taurus* FSH, the present invention is not limited to the use of said sequence and the alpha subunit sequence of the of any other ungulate may be used. In turn, the spacer sequence SEQ ID No. 3 may come from any other sequence or be any spacer sequence known in the state of the art, with the condition that said sequence is sufficiently flexible to allow the correct folding of the two subunits of the single-chain recombinant bovine FSH.

The sequence variants of SEQ ID No. 1, SEQ ID No. 2 and SEQ ID No. 3, may have an identity of at least 80% with the original sequence, preferably having a sequence identity of at least 85%, even more preferably at least 95% identity, where, regardless of the variations that the original sequences may suffer, they maintain their biological function.

The first mentioned synthetic polynucleotide sequence encoding the beta subunit of the follicle-stimulating hormone of the *Bos taurus* species having sequence SEQ ID No. 1, includes at its 5' end a sequence encoding an endogenous secretion signal, although in another preferred embodiment the polynucleotide sequence could have a heterologous signal secretion. When the polynucleotide sequence of the present invention is transduced into a cell through an expression vector, said signal sequence allows its secretion into the medium to be easily purified. In particular, the nucleotide sequence encoding that endogenous secretion signal corresponds to the first 54 bases of SEQ ID No. 1.

On the other hand, the second polynucleotide encoding the alpha subunit of the follicle-stimulating hormone of the *Bos taurus* species having sequence SEQ ID No. 2, includes at its 3' end a sequence encoding a multiple histidine region, although in another preferred embodiment the polynucleotide sequence may not have said region, or have another type of region like that of the Strep-tag® system. In particular, this region comprises between 6 and 10 histidine, preferably 6 histidine, and it is used for the purification of the single-chain recombinant bovine FSH. Particularly, the nucleotide sequence encoding this region corresponds to the last 27 bases of SEQ ID No. 2.

In a preferred embodiment, the synthetic polynucleotide sequence encoding a single-chain recombinant bovine follicle-stimulating hormone is the sequence identified as SEQ ID No. 4. This sequence is formed by the linking of the nucleotide sequences SEQ ID No. 1, SEQ ID No. No. 2, and SEQ ID No. 3, which include the nucleotide sequence encoding the endogenous secretion signal that forms part of SEQ ID No. 1, and the sequence encoding the histidine region that forms part of SEQ ID No. 2.

A second object of the present invention is a vector for the expression of a synthetic polynucleotide sequence encoding a single-chain recombinant bovine follicle-stimulating hormone, comprising a DNA construct containing a promoter sequence operatively linked to:
(i) a first polynucleotide encoding the beta subunit of the follicle-stimulating hormone of the *Bos taurus* species having sequence SEQ ID No. 1, or a variant thereof with at least 85% sequence identity therewith, more preferably at least 95% sequence identity;
(ii) a second polynucleotide encoding the alpha subunit of the follicle-stimulating hormone of the *Bos taurus* species having sequence SEQ ID No. 2 or a variant thereof, with at least 85% sequence identity therewith, more preferably at least 95% sequence identity; and
(iii) a third polynucleotide linking said first and second polynucleotides, which encodes a spacer sequence derived from human keratin 10 having sequence SEQ ID No. 3 or a variant thereof, with at least 85% sequence identity therewith, more preferably at least 95% sequence identity; and
(iv) a cleavage and polyadenylation sequence.

Particularly, in a preferred embodiment the aforementioned vector is a lentiviral vector, but a plasmid vector or any expression vector known in the state of the art for these purposes can be used.

In a preferred embodiment, the vector for the expression of a synthetic polynucleotide sequence encoding a single-chain recombinant bovine follicle-stimulating hormone, comprises a DNA construct containing a promoter sequence operatively linked to SEQ ID No. 4 and a cleavage and polyadenylation sequence. In a preferred embodiment, the DNA construct also includes a protein for the detection of the expression, preferably in that case the green fluorescent protein (GFP) is used, but not limited to this particular marker.

The nucleotide sequences encoding SEQ ID No. 4 and the sequence encoding the green fluorescent protein are found under the same cytomegalovirus immediate early promoter, although any other suitable promoter from those already known in the art may be used.

In this preferred embodiment, the nucleotide sequence encoding the green fluorescent protein is used only to detect the level of expression, to select by fluorescence those clones of transduced cells that express the highest levels of the protein of interest, in this particular case, the single-chain recombinant bovine FSH having SEQ ID No. 4.

Subsequently, and with standard laboratory methodology, the green fluorescent protein is eliminated in order to obtain only the single-chain recombinant bovine FSH.

A third object of the present invention is a transduced cell expressing a single-chain recombinant bovine follicle-stimulating hormone, comprising a vector for the expression of a synthetic polynucleotide sequence encoding said single-chain recombinant bovine follicle-stimulating hormone, wherein said vector comprises a DNA construct that contains a promoter sequence operatively linked to:
(i) a first polynucleotide encoding the beta subunit of the *Bos taurus* follicle-stimulating hormone having sequence SEQ ID No. 1, or a variant thereof with at least 85% sequence identity therewith;
(ii) a second polynucleotide encoding the alpha subunit of the *Bos taurus* follicle-stimulating hormone having sequence SEQ ID No. 2 or a variant thereof with at least 85% sequence identity therewith; and
(iii) a third polynucleotide linking said first and second polynucleotides, which encodes a spacer sequence derived from human keratin 10 having sequence SEQ ID No. 3 or a variant thereof with at least 85% sequence identity therewith;
(iv) a cleavage and polyadenylation sequence.

In a preferred embodiment, the transduced cell is a Chinese hamster ovary cell, although the invention is not limited to this host cell. The possibility of transduction of other cell types is considered, such as: HEK 293, NS0, BHK-21, PER.C6 or U937. Moreover, any recombinant protein expression system can be used, such as expression system in insect cells, yeast, algae, genetically modified animals or even in cell-free systems.

In a preferred embodiment, the cell transduced with the vector for the expression of a synthetic polynucleotide sequence encoding a single-chain recombinant bovine follicle-stimulating hormone, comprises a DNA construct containing a promoter sequence operatively linked to SEQ ID No. 4 and a cleavage and polyadenylation sequence. Preferably, the expression vector used for the transduction of the cells is the lentiviral expression vector previously mentioned.

A fourth object of the present invention is a synthetic polypeptide sequence of a single-chain recombinant bovine follicle-stimulating hormone, comprising:
(i) a first polypeptide encoding the beta subunit of the *Bos taurus* follicle-stimulating hormone having sequence SEQ ID No. 5, or a variant thereof with at least 85% sequence identity therewith;
(ii) a second polypeptide encoding the alpha subunit of the *Bos taurus* follicle-stimulating hormone having sequence SEQ ID No. 6 or a variant thereof with at least 85% sequence identity therewith; and
(iii) a third polypeptide linking said first and second polypeptides, which is a spacer sequence derived from human keratin 10 having sequence SEQ ID No. 7 or a variant thereof with at least 85% sequence identity therewith, comprising at least 2 N-glycosylation sites.

The variants of the sequences SEQ ID No. 5, SEQ ID No. 6, and SEQ ID No. 7, can have an identity of at least 80% with the original sequence, where preferably they have a sequence identity of at least 85%, even more preferably at least 95% identity, where, regardless of the variations that the original sequences suffer, they maintain their biological function.

While it is mentioned that preferably SEQ ID No. 5 corresponds to the beta subunit of the beta subunit sequence of the *Bos taurus* FSH, the present invention is not limited to the use of said sequence, and the beta subunit sequence of any other ungulate may be used. Similarly, while the preferred embodiment of the invention is SEQ ID No. 6 that corresponds to the alpha subunit of the alpha subunit sequence of the *Bos Taurus* FSH, the present invention is not limited to the use of said sequence, and the alpha subunit sequence of any other ungulate may be used. In turn, the spacer sequence SEQ ID No. 7 can come from any other sequence or be any spacer sequence known in the state of the art, with the condition that said sequence is sufficiently flexible to allow the correct folding of the two subunits of the single-chain recombinant bovine FSH, and that it contains at least 2 sites of N-glycosylation.

Preferably, the first polypeptide having sequence SEQ ID No. 5 includes at its amino terminal end a signal sequence of endogenous secretion, although the presence of said signal sequence is not necessary. In another embodiment of the present invention, SEQ ID No. 5 may not contain said signal sequence since it is not relevant for obtaining the single-chain recombinant bovine FSH. In turn, the second polypeptide having sequence SEQ ID No. 6 includes at its carboxyl terminal end a multiple histidine region, although in another preferred embodiment the polynucleotide sequence may not have said region or have another type of region, such as that of the system Strep-tag®, which comprises 8 specific amino acids that give the protein a particular protein purification method.

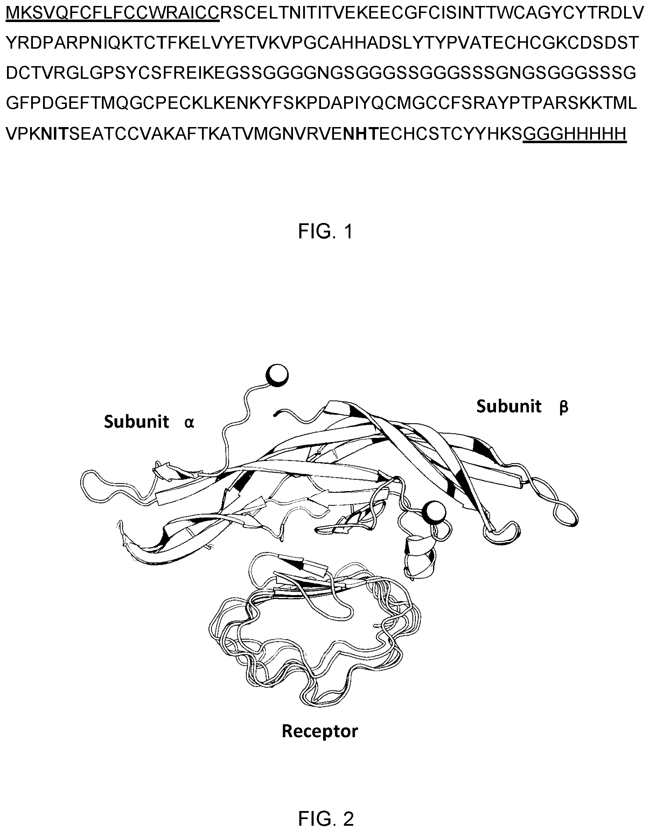

In a preferred embodiment, the synthetic polypeptide sequence of a single-chain recombinant bovine follicle-stimulating hormone is the sequence SEQ ID No. 8, as shown in FIG. 1. The underlined amino acids at the amino terminal end correspond to the sequence of endogenous secretion signal peptide, while the underlined amino acids at the carboxy terminal end correspond to the histidine region. In this preferred embodiment, the N-glycosylation sites that include the peptide of the spacer sequence, which allow to increase the half-life of this recombinant protein, are shown in bold in FIG. 1. This preferred embodiment allows, on the one hand, that the single-chain recombinant bovine FSH be expressed and secreted into the culture medium, which facilitates its collection, and on the other hand, the histidine region allows the protein obtained to be easily purified by means of metal ion affinity chromatography.

On the other hand, the preferred embodiment of the single-chain recombinant bovine FSH of SEQ ID No. 8 has a three-dimensional structure as observed in FIG. 2, which demonstrates that, despite being of single chain, this recombinant protein is capable of folding in the same way as the natural heterodimeric protein.

A fifth object of the present invention is a method to obtain a single-chain recombinant bovine follicle-stimulating hormone, comprising the steps of:
- providing a synthetic polynucleotide sequence encoding the single-chain recombinant bovine follicle-stimulating hormone, comprising:
   (i) a first polynucleotide encoding the beta subunit of the *Bos taurus* follicle-stimulating hormone having sequence SEQ ID No. 1, or a variant thereof with at least 85% sequence identity therewith;
   (ii) a second polynucleotide encoding the alpha subunit of the *Bos taurus* follicle-stimulating hormone having sequence SEQ ID No. 2 or a variant thereof with at least 85% sequence identity therewith; and
   (iii) a third polynucleotide linking said first and second polynucleotides, which encodes a spacer sequence derived from human keratin 10 having sequence SEQ ID No. 3 or a variant thereof with at least 85% sequence identity therewith;
- cloning said synthetic polynucleotide sequence into an expression vector comprising a DNA construct containing a promoter sequence operatively linked to the synthetic polynucleotide sequence, a cleavage and polyadenylation sequence;
- transducing cells with said expression vector and culturing said transduced cells in a culture medium;
- obtaining said culture medium containing the synthetic polypeptide sequence of the single-chain recombinant bovine follicle-stimulating hormone produced by the transduced cells; and
- purifying the synthetic polypeptide sequence of the single-chain recombinant bovine follicle-stimulating hormone from the culture medium.

Preferably, the synthetic polynucleotide sequence encoding the single-chain recombinant bovine follicle-stimulating hormone is SEQ ID No. 4, and the synthetic polypeptide sequence of the single-chain recombinant bovine follicle-stimulating hormone is SEQ ID No. 8.

In another preferred embodiment, the expression vector used in the aforementioned method is a lentiviral vector.

Preferably, the cells that are transduced are Chinese hamster ovary cells, in a medium preferably free of serum and free of proteins.

The purification of the synthetic polypeptide sequence of the single-chain recombinant bovine follicle-stimulating hormone is performed, in a preferred embodiment, by means of metal ion affinity chromatography. However, any purification method that is known in the state of the art may be used, without these polypeptide sequences necessarily containing a histidine region at their carboxy terminal end. That is, protein purification techniques such as isoelectric point separation, pH, or an ion exchange column may be used. They can also be separated by size or molecular weight via exclusion chromatography or SDS-PAGE, or be separated by polarity or hydrophobicity through high-performance liquid chromatography or reversed-phase chromatography.

A sixth object of the present invention is a pharmaceutical composition comprising a synthetic polynucleotide of a single-chain recombinant bovine follicle-stimulating hormone, wherein said hormone has an amino acid sequence comprising:
(i) a first polypeptide encoding the beta subunit of the *Bos taurus* follicle-stimulating hormone having sequence SEQ ID No. 5, or a variant thereof with at least 85% sequence identity therewith;
(ii) a second polypeptide encoding the alpha subunit of the *Bos taurus* follicle-stimulating hormone having sequence SEQ ID No. 6 or a variant thereof with at least 85% sequence identity therewith; and
(iii) a third polypeptide linking said first and second polypeptides, which is a spacer sequence derived from human keratin 10 having sequence SEQ ID No. 7 or a variant thereof with at least 85% sequence identity therewith, comprising at least 2 N-glycosylation sites; and pharmaceutically acceptable excipients.

Preferably, the pharmaceutical composition comprises the synthetic polypeptide sequence of the single-chain recombinant bovine follicle-stimulating hormone of SEQ ID No. 8.

The pharmaceutically acceptable excipients present in the pharmaceutical composition are carriers that enable the administration of the active compound, in this case the single-chain recombinant bovine FSH, to an ungulate. Said excipients will depend directly on the route of administration used and on the pharmacodynamic and pharmacokinetic properties intended for the composition. As such, a pharmaceutically acceptable excipient can be a binder, diluent, disintegrant, lubricant, or coating compound.

Another object of the present invention is a method of treatment for the superovulation of ungulates that allows to increase the reproduction thereof, comprising the administration of an effective dose of a pharmaceutical composition comprising:
(i) a first polypeptide encoding the beta subunit of the *Bos taurus* follicle-stimulating hormone having sequence SEQ ID No. 5, or a variant thereof with at least 85% sequence identity therewith;
(ii) a second polypeptide encoding the alpha subunit of the *Bos taurus* follicle-stimulating hormone having sequence SEQ ID No. 6 or a variant thereof with at least 85% sequence identity therewith; and
(iii) a third polypeptide linking said first and second polypeptides, which is a spacer sequence derived from human keratin 10 having sequence SEQ ID No. 7 or a variant thereof with at least 85% sequence identity therewith, comprising at least 2 N-glycosylation sites;
wherein said pharmaceutical composition is administered in multiple doses spaced between several hours and for several days. Preferably, the pharmaceutical composition of the present invention is administered every 12 hours, for 72 hours, preferably with administration of a dose of 500 µg.

In a preferred embodiment, the pharmaceutical composition comprises the synthetic polypeptide sequence of the single-chain recombinant bovine follicle-stimulating hormone having SEQ ID No. 8.

A final object of the present invention is a method to induce estrus synchronization in ungulates, which comprises the administration of a single dose between 20 and 200 µg of the pharmaceutical composition previously described to the animals.

The pharmaceutical composition of the present invention can be administered by any route known in the state of the art, although it is preferably administered through an intramuscular injection or an intravenous injection.

The methods of treatment of the present invention enable the induction of superovulation and estrus synchronization in ungulates, preferably in cattle, but the method can be used in a large number of species such as cows, sheep, goats, buffaloes, pigs, boars, horses, antelopes, bison, reindeer, llamas, alpacas, vicunas, among others, and not limited the aforementioned antecedents.

The following examples are intended to illustrate the invention and its preferred embodiments, but under no circumstances should they be considered to restrict the scope of the invention, which will be defined by the wording of the claims annexed hereto.

### Exemplary embodiments

### Example 1: Design and Production of single-chain recombinant bovine FSH (scbFSH)

In order to generate the single-chain recombinant bovine follicle-stimulating hormone, sequences comprising the heterodimer of FSH were selected together with a spacer or linker sequence.

The beta subunit of FSH was selected from the amino acid sequence of this *Bos taurus* protein, which corresponds to sequence SEQ ID No. 5. In its amino terminus, it has an endogenous secretion signal peptide and glycosylation sites. The alpha subunit was also obtained from the sequence of this *Bos taurus* protein, to which the endogenous secretion signal peptide was removed.

This subunit corresponds to the sequence SEQ ID No. 6. Since the FSH is a heterodimer, in order to select the spacer sequence, it was necessary to consider that both subunits should conserve enough steric release so as not to affect its folding and ability to assemble properly, as the heterodimer does. The spacer sequence should also not affect the interaction of the FSH with the receptor.

For this reason, a portion of the carboxyl terminal of human keratin 10 was selected as the spacer sequence, to which two possible sites of N-glycosylation were added in order to increase the half-life of the hormone; this sequence corresponds to SEQ ID No. 7. Subsequently, *in silico*, a linear sequence comprising the beta subunit (SEQ ID No. 5), the spacer sequence (SEQ ID No. 7), and the alpha subunit (SEQ ID No. 6) was generated. With this amino acid sequence, a DNA sequence was generated by means of *in silico* codons optimization for its expression in human cells. Codon optimization was performed by the company Genscript (http://www.genscript.com/).

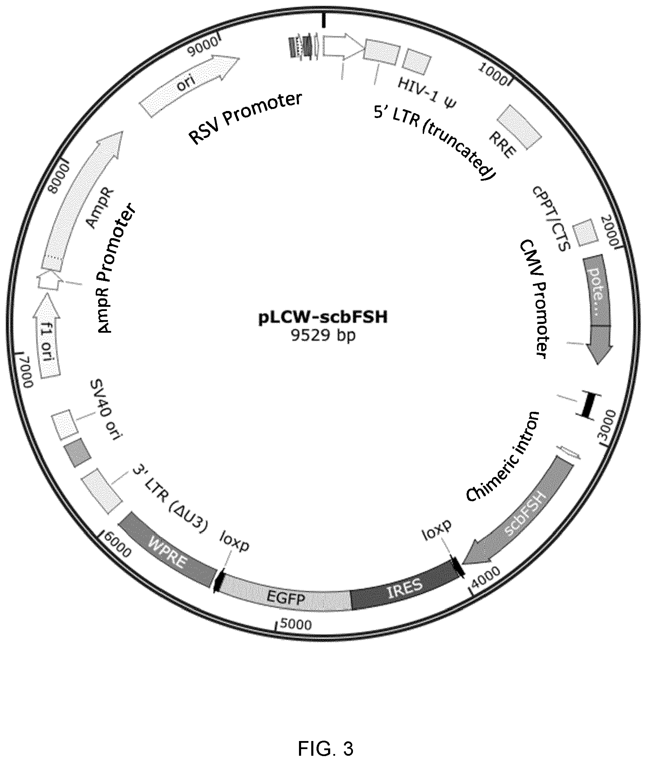

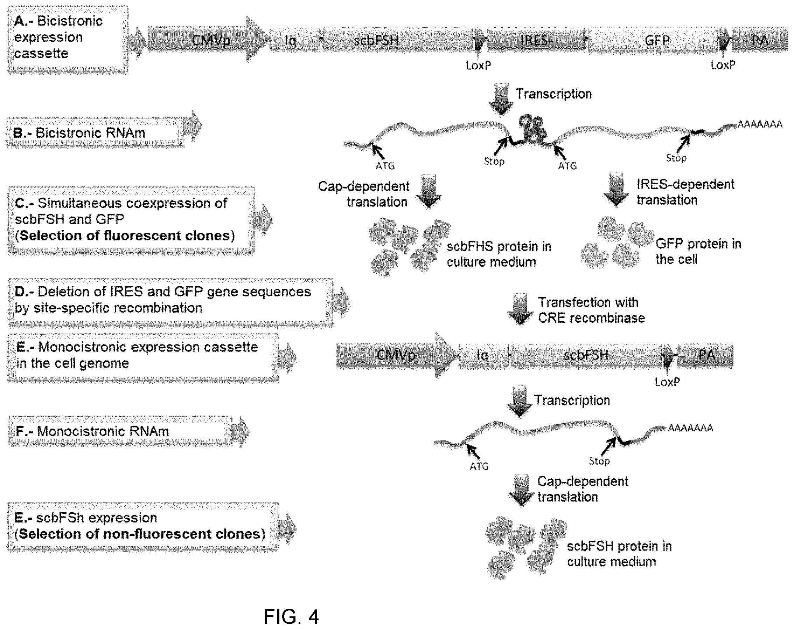

The gene sequence resulting from this process corresponds to SEQ ID No. 4, which was synthesized and cloned into the pLWC-LGL expression vector. The map of the resulting vector is shown in FIG. 3. Said vector is of lentiviral type, containing a cassette of bicistronic expression, where under the same CMV (cytomegalovirus) promoter is the scbFSH sequence and the green fluorescent protein (GFP), separated by an iRES sequence (internal ribosome entry site). The latter is flanked by LoxP sites, for its further cleavage by the CRE recombinase, after having selected the best clone for the GFP expression. With this strategy, more copies of the transgene can be incorporated into the cell genome, select the clones that produce the hormone the most based on fluorescence intensity, cleave the GFP maintaining the scbFSH expression and, subsequently, incorporate new copies of the transgene repeating the process. FIG. 4 shows a detailed scheme of the expression cassette and the biological processes of transcription, translation, and recombination involved in the production of stable clones with this cassette.

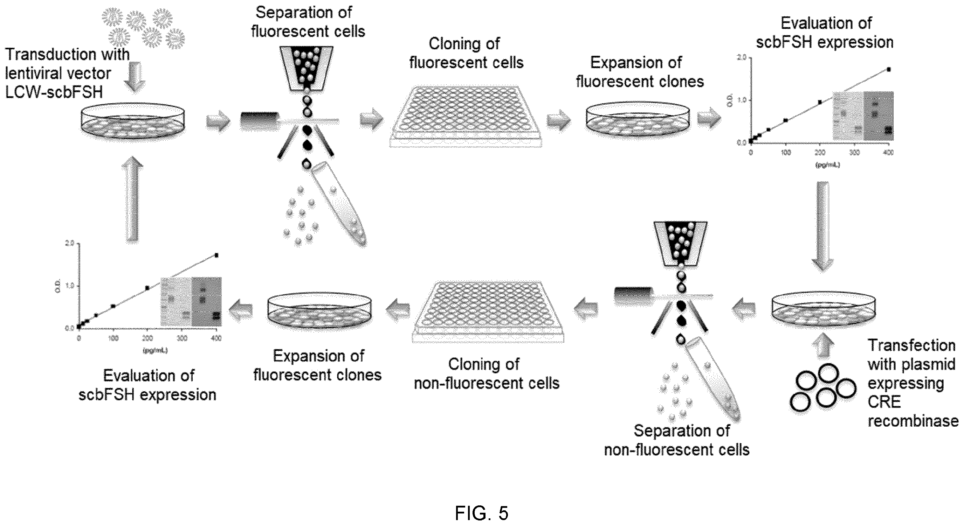

The pLCW-scbFSH vector, which already contains the cloned scbFSH sequence, was cut with the restriction enzymes EcoRI, Sail, Pst, Nael, and NgoMIV, in order to evaluate its correct cloning. In FIG. 5, the digestion pattern with each of the aforementioned enzymes is shown, in a 1% w/v agarose gel.

Having generated this vector, it was transfected in conjunction with 3 commercial helper vectors pLP1, pLP2, and pLP/VSVF, in HEK293FT cells in order to generate the lentiviral particles, using cationic polymers (polyethyleneimine, PEI). These particles were extracted from the culture medium at 48 hours post-transfection, concentrated by ultracentrifugation at 23,000 rpm for 1 hour and 30 minutes, and stored at -80°C.

Using the lentiviral particles containing the vector with the sequence for scbFSH, the transduction of the Chinese Hamster Ovary (CHO) cell line was performed, where the transduced cell populations with the highest fluorescence intensity (GFP) were selected through flow cytometry with Cell Sorter. Subsequently, each isolated clone was characterized as to the amount of scbFSH accumulated in its culture medium.

The scbFSH accumulated in the culture medium of each of the preselected clones was determined using the Bovine FSH ELISA Kit (LifeSpan BioSciences, Inc.). The results of the ELISA were taken as a basis to calculate the specific productivity (Qp) of each clone, that is, the amount of scbFSH produced by each cell in a period of 24 hours. The clone that showed better levels of expression was transfected with the CRE recombinase to cleave the coding sequence for the green fluorescent protein and was used as a basis for new rounds of transduction with the pLCW-scbFSH vector. FIG. 5 shows a general scheme of the sequential insertion procedure of transgene copies.

After three rounds of transduction/recombination, four clones were preselected for scbFSH expression analysis. The specific productivity of each of these clones is shown in Table 1.

**Table 1: ScbFSH production per day for each clone analysed**

| Clone | scbFSH (pg/cell per day) |
|---|---|
| 1 | 32 |
| 2 | 21 |
| 3 | 30 |
| 4 | 19 |

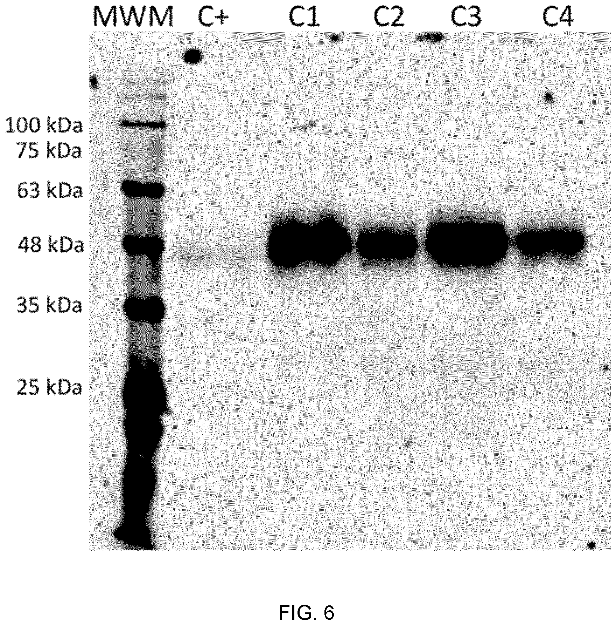

FIG. 6 shows a *Western blot* assay where the scbFSH contained in 100 microlites of the conditioned medium of the selected clones is detected. With the results of the ELISA and the *Western blot,* Clone 1 was selected to continue to the following stages of scale-up and purification of scbFSH. This clone was called CHO-scbFSH.

The CHO-scbFSH clone was adapted to growth conditions in a serum free HyClone SFM4CHO medium. The culture conditions were established in 250 mL spinner flasks using temperatures between 35 and 37°C in a SFM4CHO medium supplemented with 4mM glutamine. The cells adapted to suspension were transferred to a 10-litre scale bioreactor (Winpact fermentation system, FS-07 series). Cultures were kept at 35°C with an average cell density of 2x10⁶ cells/mL, and a perfusion rate of 1 volume per day.

The collected medium was clarified by means of sequential filtration through 5 and 0.4 micrometres. The clarified medium was stored at -80°C until further purification by means of metal ion affinity chromatography (IMAC).

### Example 2: Purification of bovine FSH

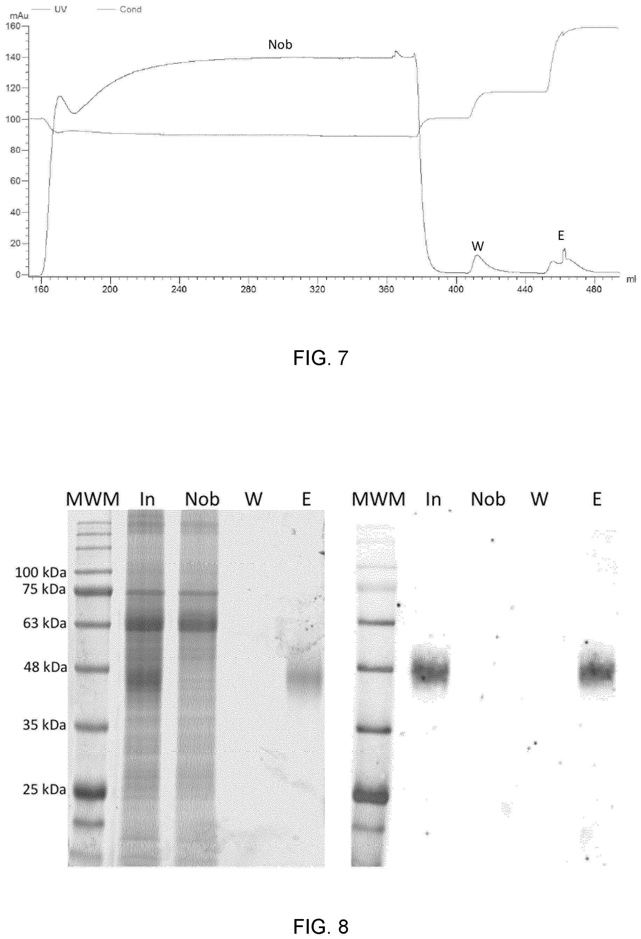

The scbFSH contained in the culture medium was purified by means of metal ion affinity chromatography using a Ni Sepharose High Performance matrix, on an XK 50/20 column (GE healthcare). The chromatography was performed on an AKTA PRIME PLUS (GE Healthcare). The input medium was supplemented with 30 mM imidazole and adjusted to pH7.4. In order to separate contaminating proteins, the column was washed with 100 mM imidazole in phosphate buffer. The elution was carried out at 200 mM imidazole. FIG. 7 shows the chromatogram of the purification. The peak marked with the letter E corresponds to the eluted fraction where the scbFSH of the present invention is found in its purified form. SDS-PAGE and *Western Blot* assays were performed to detect the presence of the hormone in each of the fractions obtained in the purification (FIG 8). It was possible to corroborate that the entire scbFSH eluted at 200 mM imidazole. No losses of the hormone in the fraction corresponding to the non-bound proteins (Nob) were observed, nor in the washing fraction (W).

The purified scbFSH was diafiltered against phosphate buffer, quantified, and lyophilized in glass flasks for later uses.

### Example 3: Analysis of the oligosaccharide profile of purified scbFSH

In correspondence with its amino acid sequence, the scbFSH molecule should have a molecular weight of 26.6 kDa. However, this molecule has a total of six potential N-glycosylation sites. Four of these sites are endogenous, while the remaining two were incorporated as part of the spacer or linker sequence linking the alpha and beta chains. The N-glycans incorporated to these sites are usually of heterogeneous size and composition, providing an additional weight between 3.5 and 5 kDa each. Thus, if it is considered that the 6 N-glycosylation sites have been occupied, then, the sum of the weights contributing as N-glycosylation should oscillate between 21 and 30 kDa, generating a molecule having a heterogeneous molecular weight between 48 and 57 kDa. The SDS-PAGE and *Western Blot* assays show that, indeed, scbFSH migrates as a diffuse band above 48 kDa, suggesting that the 6 potential N-glycosylation sites could be occupied (Figs 6 and 8).

In order to analyse its oligosaccharide profile, the scbFSH molecule was digested with the PNGase F enzyme. The released oligosaccharides were separated by means of normal-phase liquid chromatography (NP-HPLC) on a TSK-Gel Amide-80 column (Tosoh Biosep, Japan). These oligosaccharides were also subjected to digestion with the neuraminidase enzyme in order to remove the terminal sialic acids and thus determine the degree of *sialylation* of the N-linked sugars.

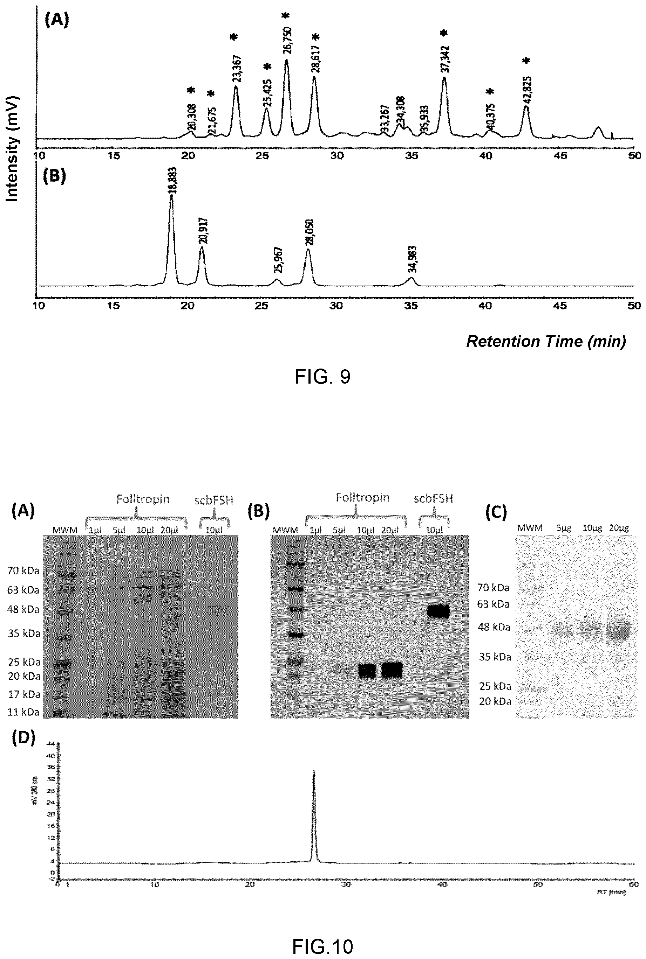

FIG. 9 shows the chromatograms of the N-linked oligosaccharides before and after being digested with the neuraminidase enzyme. In section B of this figure, the disappearance of the peaks with the highest retention time is observed, which demonstrates that the oligosaccharides N-linked to the scbFSH (section A) are highly sialylated. The degree of sialylation of a glycoprotein is relevant, since the sialic acid at the ends of the oligosaccharide chains prevents the molecule from being attracted by the mannose and galactose receptors of the liver, thus protecting the molecule from hepatic metabolization.

### Example 4: Comparison of scbFSH with commercial FSHs that are currently used in bovines.

The current market of the FSH for the agricultural and livestock sector is dominated by two fundamental commercial products: Pluset (25%) and Folltropin-V (70%). However, these products, as it was previously explained, are extracted from the pituitary gland of pigs, with the consequent disadvantages linked to them. Since they are the only products available to conduct the relevant comparative analyses, in order to demonstrate the viability of the scbFSH of the present invention as an alternative, the following studies were carried out:

### a) Purity and electrophoretic mobility

The purity and electrophoretic mobility of scbFSH was evaluated in comparison with its commercial pair Folltropin-V®. Both molecules were subjected to SDS-PAGE and *Western blot* assays.

FIG. 10 shows the comparison of the scbFSH purity analysis with respect to commercial FSH Folltropin-V®. **(A)** 10% SDS PAGE, **(B)** *Western blot* using an anti-beta chain antibody of FSH. For A and B, variable amounts of the commercial hormone Folltropin-V® (1, 5, 10, 20 µl), and a fixed amount of 10 µl of the scbFSH were applied to the gel. Both hormones were at the same concentration (approximately 100 µg/mL). **(C)** 10% SDS PAGE, where 5, 10 and 20 µg of the scbFSH are applied. **(D)** Determination of scbFSH purity by means of HPLC gel-filtration chromatography. HPLC chromatography shows a single peak demonstrating the high level of purity of the sample.

The SDS-PAGE demonstrated that the scbFSH produced by recombinant DNA technology is a highly pure product, which could also be evidenced by means of HPLC gel-filtration chromatography, where a single peak could be observed. In the *Western blot* assay, Folltropin-V® shows an immunoreactive band of approximately 23 kDa, resulting from the dissociation of the alpha and beta chains, whereas the scbFSH shows an immunoreactive band of around 50 kDa corresponding to the sum of weight weights of the alpha and beta chains of bovine FSH (*approximately* 26.6 *kDa*), plus an additional 21 to 30 kDa for N-glycosylation.

### b) Biological Activity (in vitro maturation of bovine oocytes)

The biological activity of scbFSH produced by recombinant DNA technology was evaluated in an *in vitro* maturation assay of bovine oocytes. In this assay, the commercial counterparts Folltropin-V® and Pluset® were also evaluated. To that end, the oocytes were aspirated from ovaries of cows slaughtered in a slaughterhouse. After centrifuging at 3000g for 10 minutes, the oocytes with cumulus from at least five cell layers were selected and washed three times in a washing medium (Tyrode's medium with HEPES buffer solution and supplemented with 0.3% BSA, 0.2 mM sodium pyruvate, and 50 µg/mL gentamicin). Once washed, groups of 25 oocytes were transferred to 100 µl drops of maturation medium (SOF supplemented with 0.8% BSA, 0.33 mM sodium pyruvate, 1X MEM essential amino acids, 1X MEM non-essential amino acids, 1 mM of glutamine, 5 µg/mL LH, 1 µg/mL 17b-estradiol, and 50 µg/mL gentamicin), supplemented, or not, with FSH. The oocytes were cultured for 24 hours at 38.5°C, in 5% CO₂, and 95% humidity. After this time, the oocytes were analysed by microscopy and the expansion of the cumulus was taken as a FSH-induced maturation criterion. FIG. 16 shows the results obtained in the present study, where the following controls were used: **(A)** maturation medium without FSH, **(B)** maturation medium containing 1µg/mL FSH Folltropin-V®, **(C)** maturation medium containing 0.01 IU/mL Pluset®, **(D)** maturation medium containing 1.5 ng/mL scbFSH.

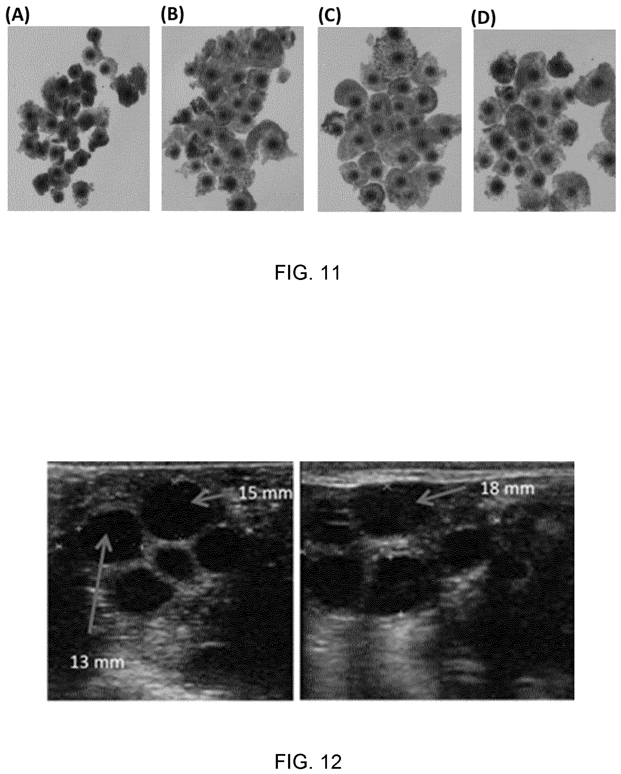

The expansion of the cumulus-oocyte complexes, as well as the extrusion of the first polar body, were taken as a maturation criterion. This assay showed that all three hormones are biologically active, in terms of their ability to induce *in vitro* maturation of bovine oocytes (FIG 11). There were no significant differences in the maturation rates between the hormones.

### c) Biological Activity (Induction of superovulation in ovines)

The biological activity of scbFSH produced by recombinant DNA technology was also evaluated by means of a superovulation assay in ovines. The commercial counterparts Folltropin-V® and Pluset® were also evaluated in the assay, following consensus protocols for each hormone as described in the table below. The assay was conducted on Corriedale sheep between 50 and 60 kg, which were randomized into three experimental groups according to the three types of hormones evaluated. The different FSH were administered intramuscularly at 12-hour intervals in decreasing doses for 96 hours, starting 24 hours before the removal of the intravaginal sponge containing progestins (60 mg medroxyprogesterone acetate, Progespon®, Syntex, Argentina). At the end of the scheme, transrectal ultrasonography was performed and the follicles larger than 10 mm in diameter were counted. The results shown in the table below demonstrate that, under the conditions evaluated, the scbFSH hormone yields better superovulation levels than its commercial counterparts Folltropin-V® and Pluset®.

**Table 2. Results of the superovulation assay in sheep using different FSHs**

| Hormone | **Animal** | **Dose/Scheme** | **Follicle No.** | **Mean ± Standard Dev.** |
|---|---|---|---|---|
| Folltropin-V® | 1 | Total dose of 200 mg NIH-FSH-P1 | 10 | 13.6 ± 3.6 |
| Folltropin-V® | 2 | | 11 | |
| | | 8 applications (*every 12 h*) on a decreasing basis as follows: 40, 40, 30, 30, 20, 20, 10 and 10 mg. | | |
| Folltropin-V® | 3 | | 19 | |
| Folltropin-V® | 4 | | 13 | |
| Folltropin-V® | 5 | | 15 | |
| Pluset® | 6 | Total dose of 300 UI | 13 | 10.8 ± 3.7 |
| Pluset® | 7 | 8 applications (*every 12 h*) on a decreasing | 7 | |
| Pluset® | 8 | | 16 | |
| Pluset® | 9 | basis as follows: 60, 60, 45, 45, 30, 30, 15 and 15 IU. | 10 | |
| Pluset® | 10 | | 8 | |
| scbFSH | 11 | Total dose of 250 µg | 15 20 | 17 ± 2.9 |
| scbFSH | 12 | 8 applications (*every 12 h*) on a decreasing basis as follows: 50, 50, 37.5, 37.5, 25, 25, 12.5 and 12.5 µg. | | |
| scbFSH | 13 | | 18 | |
| scbFSH | 14 | | 13 | |
| scbFSH | 15 | | 19 | |

The results shown in Table 2 demonstrate that, under the conditions evaluated, the scbFSH hormone yields better superovulation levels than its commercial counterparts Folltropin-V® and Pluset®.

### Example 5: Superovulation and estrus synchronization assay in cattle.

The ability of scbFSH to induce superovulation in bovine cattle was evaluated in five red Holstein Friesian cows older than 5 years of age with monitoring of estrous cycle prior to the field test, determining an average of 20 days for the animals under study, and maintaining ad *libitum* feeding in clover and ryegrass meadow.

A superovulation scheme with scbFSH of 96 hours was followed, starting on day 4 after implanting a bovine intravaginal device impregnated with progesterone (Syntex). In the superovulation scheme, a total dose of 500 µg scbFSH divided into 8 doses of 62.5 µg each was administered at 12-hour intervals. On day 7, 5 ml of Lutalyse (Zoetis) were administered, and on day 9 transrectal ultrasonography was performed to count the total number of follicles larger than 10 mm in diameter, both on the left ovary (LO) and the right ovary (RO). The results of this assay are shown in Table 3 below, showing the number of follicles per ovary, and per animal 24 hours after completing the hormonal induction.

**Table 3. Results of superovulation assay in sheep using scbFSH**

| Cow 1 | | Cow 2 | | Cow 3 | | Cow 4 | | Cow 5 | |
|---|---|---|---|---|---|---|---|---|---|
| **LO** | **RO** | **LO** | **RO** | **LO** | **RO** | **LO** | **RO** | **LO** | **RO** |
| 9 | 11 | 10 | 13 | 9 | 11 | 13 | 12 | 11 | 14 |
| **22.6** ± **2.5 follicles/animal** | | | | | | | | | |

The superovulation assay in bovines yielded an average ovarian response of 22.6 ± 2.5 follicles larger than 10 mm. This type of response is significantly superior to the response usually obtained with commercial hormones, where the number of follicles ranges between 10 to 17 per animal.

The ovulatory response in bovines also constitutes evidence of the ability of the hormone scbFSH to induce estrus synchronization as a previous step to fixed-time artificial insemination (FTAI), demonstrating the potential of this hormone for assisted reproduction applications where it is necessary to synchronize the period when births occur.

FIG. 12 shows an illustrative image of the follicles detected by means of transrectal ultrasonography and the size thereof.

### Advantages of the proposed technical solution

The solution proposed by the present invention is a viable FSH alternative for use in bovine cattle, which would complement the products currently on the market, additionally offering the following advantages:
(1) The risk of contamination with pathogens is avoided: Because they originate from slaughtered animals, commercial products have the risk of being contaminated with pathogens or prions. In the case of scbFSH, being a recombinant product, this risk does not exist.
(2) There is no contamination with luteinizing hormone (LH): All FSH obtained from the pituitary gland have the LH molecule as a contaminant. Some of these preparations even declare the effect of LH. The LH content affects the efficiency of the superovulation process, affects the efficiency of fertilization, and also affects the quality of the embryos. In the case of scbFSH, being a recombinant product, it would not have this contaminant.
(3) Extended circulation half-life: The FSH molecules currently on the market have a circulation half-life of about 5 hours. This short half-life requires establishing a superovulation protocol that involves eight injections (two injections per day, for 4 days). The average circulation half-life of the scbFSH is about 10-13 hours, which allows establishing superovulation protocols of a single daily dose for 4 days.
(4) Low risk of inducing bovine immune response: Due to its porcine origin, the current FSH molecules can induce a humoral immune response when administered in other species (including bovines). In the case of the scbFSH, due to its bovine origin, it has no possibility of inducing an immune response when used in this species.

## Claims

1. A synthetic polynucleotide sequence encoding a single-chain recombinant bovine follicle-stimulating hormone, **CHARACTERIZED in that** it comprises:
(i) a first polynucleotide encoding the beta subunit of the *Bos taurus* follicle-stimulating hormone having sequence SEQ ID No. 1, or a variant thereof with at least 85% sequence identity therewith;
(ii) a second polynucleotide encoding the alpha subunit of the *Bos taurus* follicle-stimulating hormone having sequence SEQ ID No. 2 or a variant thereof with at least 85% sequence identity therewith; and
(iii) a third polynucleotide linking said first and second polynucleotides, which encodes a spacer sequence derived from human keratin 10 having sequence SEQ ID No. 3 or a variant thereof with at least 85% sequence identity therewith.

2. The synthetic nucleotide sequence of claim 1, **CHARACTERIZED in that** the first polynucleotide having sequence SEQ ID No. 1 includes at its 5' end a sequence encoding an endogenous secretion signal.

3. The synthetic nucleotide sequence of claim 1, **CHARACTERIZED in that** the second polynucleotide having sequence SEQ ID No. 2 includes at its 3' end a sequence encoding a multiple histidine region.

4. The synthetic polynucleotide sequence of claim 1, **CHARACTERIZED in that** it is the sequence SEQ ID No. 4.

5. A vector for the expression of a synthetic polynucleotide sequence encoding a single-chain recombinant bovine follicle-stimulating hormone, **CHARACTERIZED in that** it comprises a DNA construct containing:
a) a promoter sequence operatively linked to:
(i) a first polynucleotide encoding the beta subunit of the *Bos taurus* follicle-stimulating hormone having sequence SEQ ID No. 1, or a variant thereof with at least 85% sequence identity therewith;
(ii) a second polynucleotide encoding the alpha subunit of the *Bos taurus* follicle-stimulating hormone having sequence SEQ ID No. 2 or a variant thereof with at least 85% sequence identity therewith;
(iii) a third polynucleotide linking said first and second polynucleotides, which encodes a spacer sequence derived from human keratin 10 having sequence SEQ ID No. 3 or a variant thereof with at least 85% sequence identity therewith; and
b) a cleavage and polyadenylation sequence.

6. The vector of claim 5, **CHARACTERIZED in that** said vector is a lentiviral vector.

7. A transduced cell expressing a single-chain recombinant bovine follicle-stimulating hormone, CHARACTERIZED because it comprises a vector for the expression of a synthetic polynucleotide sequence encoding said single-chain recombinant bovine follicle-stimulating hormone, wherein said vector comprises a DNA construct containing:
a) a promoter sequence operatively linked to:
(i) a first polynucleotide encoding the beta subunit of the *Bos taurus* follicle-stimulating hormone having sequence SEQ ID No. 1, or a variant thereof with at least 85% sequence identity therewith;
(ii) a second polynucleotide encoding the alpha subunit of the *Bos taurus* follicle-stimulating hormone having sequence SEQ ID No. 2 or a variant thereof with at least 85% sequence identity therewith;
(iii) a third polynucleotide linking said first and second polynucleotides, which encodes a spacer sequence derived from human keratin 10 having sequence SEQ ID No. 3 or a variant thereof with at least 85% sequence identity therewith; and
b) a cleavage and polyadenylation sequence.

8. The transduced cell of claim 7, **CHARACTERIZED in that** it is a Chinese hamster ovary cell.

9. A synthetic polypeptide sequence of a single-chain recombinant bovine follicle-stimulating hormone, **CHARACTERIZED in that** it comprises:
(i) a first polypeptide encoding the beta subunit of the *Bos taurus* follicle-stimulating hormone having sequence SEQ ID No. 5, or a variant thereof with at least 85% sequence identity therewith;
(ii) a second polypeptide encoding the alpha subunit of the *Bos taurus* follicle-stimulating hormone having sequence SEQ ID No. 6 or a variant thereof with at least 85% sequence identity therewith;
(iii) a third polypeptide linking said first and second polypeptides, which is a spacer sequence derived from human keratin 10 having sequence SEQ ID No. 7 or a variant thereof with at least 85% sequence identity therewith, comprising at least 2 N-glycosylation sites.

10. The synthetic polypeptide sequence of claim 9, **CHARACTERIZED in that** the first polypeptide having sequence SEQ ID No. 5 includes at its amino terminal end an endogenous secretion signal sequence.

11. The synthetic polypeptide sequence of claim 9, **CHARACTERIZED in that** the second polypeptide having sequence SEQ ID No. 6 includes at its carboxyl-terminal end a multiple histidine region.

12. The synthetic polypeptide sequence of claim 9, **CHARACTERIZED in that** it is the sequence SEQ ID No. 8.

13. A method for obtaining a simple chain bovine recombinant follicle-stimulating hormone, **CHARACTERIZED in that** it comprises the steps of:
- providing a synthetic polynucleotide sequence encoding a single-chain recombinant bovine follicle-stimulating hormone, comprising:
(i) a first polynucleotide encoding the beta subunit of the *Bos taurus* follicle-stimulating hormone having sequence SEQ ID No. 1, or a variant thereof with at least 85% sequence identity therewith;
(ii) a second polynucleotide encoding the alpha subunit of the *Bos taurus* follicle-stimulating hormone having sequence SEQ ID No. 2 or a variant thereof with at least 85% sequence identity therewith;
(iii) a third polynucleotide linking said first and second polynucleotides, which encodes a spacer sequence derived from human keratin 10 having sequence SEQ ID No. 3 or a variant thereof with at least 85% sequence identity therewith;
- cloning said synthetic polynucleotide sequence encoding a single-chain recombinant bovine follicle-stimulating hormone into an expression vector comprising a DNA construct containing a promoter sequence operatively linked to said synthetic polynucleotide sequence and to a cleavage and polyadenylation sequence;
- transducing cells with said expression vector and culturing said transduced cells in a culture medium;
- obtaining from said culture medium the simple chain bovine recombinant follicle-stimulating hormone produced by the transduced cells; and
- purifying said single-chain recombinant bovine follicle-stimulating hormone obtained from the culture medium.

14. The method of claim 13, **CHARACTERIZED in that** the synthetic polynucleotide sequence encoding the single-chain recombinant bovine follicle-stimulating hormone is SEQ ID No. 4.

15. The method of claim 13, **CHARACTERIZED in that** the simple chain recombinant bovine follicle-stimulating hormone has the polypeptide sequence of SEQ ID No. 8.

16. The method of claim 13, **CHARACTERIZED in that** the expression vector is a lentiviral vector.

17. The method of claim 13, **CHARACTERIZED in that** said cells are Chinese hamster ovary cells.

18. The method of claim 13, **CHARACTERIZED in that** the culture medium is a serum-free and protein-free medium.

19. The method of claim 13, **CHARACTERIZED in that** the purification is carried out by means of metal ion affinity chromatography.

20. A pharmaceutical composition, **CHARACTERIZED in that** it comprises a synthetic polypeptide corresponding to a single-chain recombinant bovine follicle-stimulating hormone having the polypeptide sequence of claim 9, and pharmaceutically acceptable excipients.

21. A method for inducing superovulation of ungulates, **CHARACTERIZED in that** it comprises the administration of a dose of the pharmaceutical composition of claim 20 every 12 hours, for 72 hours.

22. The method of claim 21, **CHARACTERIZED in that** the total administered dose of said pharmaceutical composition is between 100 and 1000 µg.

23. A method for inducing estrus synchronization in ungulates, **CHARACTERIZED in that** it comprises administering a single dose of the pharmaceutical composition of claim 20.

24. The method of claim 23, **CHARACTERIZED in that** the total administered dose of said pharmaceutical composition is between 20 and 200 µg.


LISTADO DE SECUENCIAS

<110>  Centro de Biotecnologia y Biomedicina SpA
       Fundacao Edson Queiroz - Universidad de Fortaleza

<120>  HORMONA FOLICULO ESTIMULANTE BOVINA RECOMBINANTE, COMPOSICION QUE
       LA COMPRENDE Y METODO PARA INDUCIR SUPEROVULACION Y
       SINCRONIZACION DEL CELO EN BOVINOS UTILIZANDO DICHA HORMONA

<130>  1219-778-CL

<140>  CL 1347-2018
<141>  2018-05-18

<160>  8

<170>  PatentIn version 3.5

<210>  1
<211>  387
<212>  ADN
<213>  Bison bison

<400>  1
atgaaatctg tccaattttg tttcctcttc tgctgctggc gggctatctg ttgcaggagt      60

tgcgaactca ctaatatcac tataacagtc gagaaagaag aatgtggatt ttgtattagc     120

attaacacta cttggtgcgc aggatattgt tacacaaggg atttggtgta cagagacccc     180

gcccgcccaa acattcaaaa gacttgtacg ttcaaagaat tggtgtatga gaccgtgaag     240

gtgcctggat gcgcacacca tgctgactcc ctgtatacat accccgtagc cactgaatgc     300

cactgtggaa aatgcgactc cgactctacc gattgcactg tcagaggact tggaccctca     360

tactgctcat ttagggaaat caaagaa                                         387


<210>  2
<211>  318
<212>  ADN
<213>  Bos taurus

<400>  2
tttccggatg gcgaattcac aatgcagggt tgtcctgaat gtaaactgaa ggaaaacaag      60

tatttctcta agcctgacgc cccaatctat cagtgtatgg gctgttgttt ttccagagct     120

tacccaacac ccgctaggtc taagaaaact atgttggtgc cgaaaaatat cacatctgaa     180

gcaacttgtt gcgtggctaa agcgtttact aaggctacag tgatgggaaa cgtaagagtt     240

gaaaaccaca cagaatgcca ttgctctacc tgttactacc ataagtccgg ggggggccac      300

catcaccacc accattga                                                   318


<210>  3
<211>  99
<212>  ADN
<213>  Homo sapiens

<220>
<223>  keratina 10 C-ter

<400>  3
ggctcatctg ggggcggcgg caacggcagc ggaggcggct cttctggtgg tggatcatca      60

agtgggaatg gaagcggcgg aggcagttct tctgggggc                            99


<210>  4
<211>  804
<212>  ADN
<213>  Secuencia Artificial

<220>
<223>  rbFSH

<400>  4
atgaaatctg tccaattttg tttcctcttc tgctgctggc gggctatctg ttgcaggagt      60

tgcgaactca ctaatatcac tataacagtc gagaaagaag aatgtggatt ttgtattagc     120

attaacacta cttggtgcgc aggatattgt tacacaaggg atttggtgta cagagacccc     180

gcccgcccaa acattcaaaa gacttgtacg ttcaaagaat tggtgtatga gaccgtgaag     240

gtgcctggat gcgcacacca tgctgactcc ctgtatacat accccgtagc cactgaatgc     300

cactgtggaa aatgcgactc cgactctacc gattgcactg tcagaggact tggaccctca     360

tactgctcat ttagggaaat caaagaaggc tcatctgggg gcggcggcaa cggcagcgga     420

ggcggctctt ctggtggtgg atcatcaagt gggaatggaa gcggcggagg cagttcttct     480

gggggctttc cggatggcga attcacaatg cagggttgtc ctgaatgtaa actgaaggaa     540

aacaagtatt tctctaagcc tgacgcccca atctatcagt gtatgggctg ttgtttttcc     600

agagcttacc caacacccgc taggtctaag aaaactatgt tggtgccgaa aaatatcaca     660

tctgaagcaa cttgttgcgt ggctaaagcg tttactaagg ctacagtgat gggaaacgta     720

agagttgaaa accacacaga atgccattgc tctacctgtt actaccataa gtccgggggg     780

ggccaccatc accaccacca ttga                                            804


<210>  5
<211>  129
<212>  PRT
<213>  Bison bison

<400>  5

Met Lys Ser Val Gln Phe Cys Phe Leu Phe Cys Cys Trp Arg Ala Ile
1               5                   10                  15


Cys Cys Arg Ser Cys Glu Leu Thr Asn Ile Thr Ile Thr Val Glu Lys
            20                  25                  30


Glu Glu Cys Gly Phe Cys Ile Ser Ile Asn Thr Thr Trp Cys Ala Gly

35                  40                  45

Tyr Cys Tyr Thr Arg Asp Leu Val Tyr Arg Asp Pro Ala Arg Pro Asn
    50                  55                  60

Ile Gln Lys Thr Cys Thr Phe Lys Glu Leu Val Tyr Glu Thr Val Lys
65                  70                  75                  80

Val Pro Gly Cys Ala His His Ala Asp Ser Leu Tyr Thr Tyr Pro Val
                85                  90                  95

Ala Thr Glu Cys His Cys Gly Lys Cys Asp Ser Asp Ser Thr Asp Cys
            100                 105                 110

Thr Val Arg Gly Leu Gly Pro Ser Tyr Cys Ser Phe Arg Glu Ile Lys
        115                 120                 125

Glu

<210>  6
<211>  105
<212>  PRT
<213>  Bos taurus

<400>  6

Phe Pro Asp Gly Glu Phe Thr Met Gln Gly Cys Pro Glu Cys Lys Leu
1               5                   10                  15

Lys Glu Asn Lys Tyr Phe Ser Lys Pro Asp Ala Pro Ile Tyr Gln Cys
            20                  25                  30

Met Gly Cys Cys Phe Ser Arg Ala Tyr Pro Thr Pro Ala Arg Ser Lys
        35                  40                  45

Lys Thr Met Leu Val Pro Lys Asn Ile Thr Ser Glu Ala Thr Cys Cys
    50                  55                  60

Val Ala Lys Ala Phe Thr Lys Ala Thr Val Met Gly Asn Val Arg Val
65                  70                  75                  80

Glu Asn His Thr Glu Cys His Cys Ser Thr Cys Tyr Tyr His Lys Ser
                85                  90                  95

Gly Gly Gly His His His His His His
            100                 105

<210>  7

<211>  32
<212>  PRT
<213>  Homo sapiens

<220>
<223>  queratina 10 C-ter

<400>  7

Gly Ser Ser Gly Gly Gly Gly Asn Gly Gly Gly Gly Ser Ser Gly Gly
1               5                   10                  15

Gly Ser Ser Ser Gly Asn Gly Ser Gly Gly Gly Ser Ser Ser Gly Gly
            20                  25                  30

<210>  8
<211>  266
<212>  PRT
<213>  Secuencia Artificial

<220>
<223>  rbFSH

<400>  8
   Met Lys Ser Val Gln Phe Cys Phe Leu Phe Cys Cys Trp Arg Ala Ile
   1               5                   10                  15
   Cys Cys Arg Ser Cys Glu Leu Thr Asn Ile Thr Ile Thr Val Glu Lys
               20                  25                  30
   Glu Glu Cys Gly Phe Cys Ile Ser Ile Asn Thr Thr Trp Cys Ala Gly
           35                  40                  45
   Tyr Cys Tyr Thr Arg Asp Leu Val Tyr Arg Asp Pro Ala Arg Pro Asn
       50                  55                  60
   Ile Gln Lys Thr Cys Thr Phe Lys Glu Leu Val Tyr Glu Thr Val Lys
   65                  70                  75                  80
   Val Pro Gly Cys Ala His His Ala Asp Ser Leu Tyr Thr Tyr Pro Val
                   85                  90                  95
   Ala Thr Glu Cys His Cys Gly Lys Cys Asp Ser Asp Ser Thr Asp Cys
               100                 105                 110
   Thr Val Arg Gly Leu Gly Pro Ser Tyr Cys Ser Phe Arg Glu Ile Lys
           115                 120                 125
   Glu Gly Ser Ser Gly Gly Gly Gly Asn Gly Gly Gly Gly Ser Ser Gly
       130                 135                 140
   Gly Gly Ser Ser Ser Gly Asn Gly Ser Gly Gly Gly Ser Ser Ser Gly
   145                 150                 155                 160
   Gly Phe Pro Asp Gly Glu Phe Thr Met Gln Gly Cys Pro Glu Cys Lys
                   165                 170                 175
   Leu Lys Glu Asn Lys Tyr Phe Ser Lys Pro Asp Ala Pro Ile Tyr Gln
               180                 185                 190
   Cys Met Gly Cys Cys Phe Ser Arg Ala Tyr Pro Thr Pro Ala Arg Ser
           195                 200                 205
   Lys Lys Thr Met Leu Val Pro Lys Asn Ile Thr Ser Glu Ala Thr Cys
       210                 215                 220
   Cys Val Ala Lys Ala Phe Thr Lys Ala Thr Val Met Gly Asn Val Arg
   225                 230                 235                 240
   Val Glu Asn His Thr Glu Cys His Cys Ser Thr Cys Tyr Tyr His Lys
                   245                 250                 255
   Ser Gly Gly Gly His His His His His His
               260                 265